# EUROPEAN PATENT APPLICATION

(11) **EP 2 722 169 A1**
(43) Date of publication of application: **23.04.2014**
(21) Application number: 12800420.7
(22) Date of filing: 14.06.2012
(51) Int. Cl.: B32B 9/04, A61K 8/73, A61K 8/85, A61Q 19/00

(54) **THIN FILM SUPPORTING HYALURONIC ACID OR DERIVATIVE THEREOF AND THIN FILM COSMETIC**

(30) Priority: 14.06.2011 JP 2011132000
(71) Applicant: Shiseido Company, Ltd., Tokyo 104-0061 (JP); Nanotheta Co., Ltd., Tokyo 169-0051 (JP)
(72) Inventor: ABE, Koji, Yokohama-shi Kanagawa 224-8558 (JP); YAMAKI, Satoshi, Yokohama-shi Kanagawa 224-8558 (JP); TAKEOKA, Eriko, Yokohama-shi Kanagawa 224-8558 (JP); KIMURA, Tomoko, Yokohama-shi Kanagawa 224-8558 (JP); KUNISAWA, Naomi, Yokohama-shi Kanagawa 224-8558 (JP); TAKEOKA, Shinji, Tokyo 105-0094 (JP); FUJIE, Toshinori, Nishitokyo-shi Tokyo 188-0014 (JP); KABATA, Kohki, Yokohama-shi Kanagawa 240-0024 (JP); SAITO, Akihiro, Koga-shi Ibaraki 306-0023 (JP)
(74) Representative: Lippert, Stachow & Partner
(86) International application number: PCT/JP2012/065268
(87) International publication number: WO 2012/173198

(57) **Abstract**

The present invention provides a thin film having an excellent manageability, moisture-retaining effect, and unevenness correction effect and a thin film cosmetic.

The thin film of the present invention is a laminate of (a) a base film supporting hyaluronic acid or a derivative thereof and (b) a carrier, wherein the thickness of film (a) is 10 to 500 nm.

## Description

### RELATED APPLICATIONS

This application claims the priority of Japanese Patent Application No. 2011-132000 filed on June 14, 2011, which is incorporated herein by reference.

### FIELD OF THE INVENTION

The present invention relates to a thin film supporting hyaluronic acid or a derivative thereof and a thin film cosmetic, and in particular, relates to a thin film having an excellent manageability, moisture-retaining effect, and unevenness correction effect, a thin film cosmetic, and a beauty treatment method using the cosmetic.

### BACKGROUND OF THE INVENTION

Pack cosmetics are used to allow a film-blended drug component to effectively penetrate the skin by covering the skin with the film for a fixed time.

Examples of such pack cosmetics include a peel-off type cosmetic wherein it is peeled off after application, a wash-away type cosmetic wherein it is washed away after application, a sheet type cosmetic wherein the sheet is peeled off after application, etc.

There is no rule for the method in using pack cosmetics; however, the general method is to use them for about 10 minutes at night after the removal of makeup.

Among them, as the pack cosmetic wherein a sheet is peeled after application, for example, a sheet-like pack cosmetic containing a dextrin and a polymeric compound and having an application layer is known (for example, patent literature 1).

As the sheet for such conventional sheet-like pack cosmetics, non-woven fabric is known. However, the thickness of non-woven fabric in such pack cosmetics is large, and it has been difficult to maintain the pasted state for a long time.

On the other hand, as the thin film polymer structure, a structure having functional material on the front surface and back surface of the film is known (for example, patent literature 2). It is also known that this structure can be obtained by spin coating.

In addition, a nanosheet formed from a macromolecular ultra-thin film that is insoluble in the medium and a macromolecular film that is soluble in the medium; and a macromolecular ultra-thin film dispersion prepared therewith are known (for example, patent literature 3). However, the support of hyaluronic acid or a derivative thereof has not been mentioned.

Patent literature 1: Japanese unexamined patent publication No. 2000-302639
Patent literature 2: International unexamined patent publication No. 2008/050913
Patent literature 3: International unexamined patent publication No. 2011/046226

### DISCLOSURE OF THE INVENTION

### PROBLEM TO BE SOLVED BY THE INVENTION

The present invention was made in view of the above-described conventional art. The problem to be solved is to provide a thin film and a thin film cosmetic that are thin to the extent that there is no uncomfortable feeling when applied on the face and are usable for a long time at any time of the day or night and to provide a beauty treatment method with the use thereof.

### MEANS TO SOLVE THE PROBLEM

The present inventors have diligently studied in view of the above-described problems of the conventional arts; as a result, the present inventors have found that a thin film, which (a) a base film supporting hyaluronic acid or a derivative thereof and (b) a carrier are laminated and the thickness of film (a) is 10 to 500 nm, has an excellent manageability, moisture-retaining effect, and unevenness correction effect.

That is, the thin film of the present invention is a laminate of (a) a base film supporting hyaluronic acid or a derivative thereof and (b) a carrier, wherein the thickness of film (a) is 10 to 500 nm.

In the above-described thin film, it is preferable that the (b) carrier is a water-soluble polymer film or a cloth.

In the above-described thin film, it is preferable that the thickness of film (a) is 30 to 200 nm.

In the above-described thin film, it is preferable that the base film comprises polylactic acid, polyglycolic acid, polycaprolactone, or a copolymer thereof.

In the above-described thin film, it is preferable that the thin film is formed by the method selected from the group consisting of a spin-coating method, a gravure method, a microgravure method, and a spray-coating method.

In the above-described thin film, it is preferable that the water-soluble polymer film is selected from the group consisting of polyvinyl alcohol or a derivative thereof, a polyether or a derivative thereof, polysaccharides, and a polymer electrolyte or a salt thereof.

In the above-described thin film, it is preferable that the cloth is a non-woven fabric or a mesh.

A thin film cosmetic of the present invention is characterized by comprising the above-described thin film.

An unevenness-improving thin film cosmetic of the present invention is characterized by comprising the above-described thin film cosmetic.

A thin film cosmetic for use near the eye and near the mouth of the present invention is characterized by comprising the above-described thin film cosmetic.

A method in using the above-described thin film cosmetic of the present invention is the method wherein the water-soluble polymer film is removed by dissolution immediately before application, the obtained base film supporting hyaluronic acid or a derivative thereof is applied on the skin, so that hyaluronic acid or a derivative thereof contacts the skin, and retained for a long time.

A method in using the above-described thin film cosmetic of the present invention is the method wherein the skin is moistened, the thin film cosmetic is applied, only the cloth is peeled off, the obtained base film supporting hyaluronic acid or a derivative thereof is applied on the skin, so that hyaluronic acid or a derivative thereof contacts the skin, and is retained for a long time.

A beauty treatment method of the present invention is the method wherein the above-described thin film cosmetic is applied on the skin.

A beauty treatment method of the present invention is characterized by comprising a step of applying one or more kinds of cosmetics selected from the group consisting of lotion, milky lotion, beauty essence, and cream and a step of applying the above-described thin film cosmetic.

A cosmetic kit of the present invention is characterized by comprising the above-described thin film cosmetic and one or more kinds of cosmetics selected from the group consisting of lotion, milky lotion, beauty essence, and cream.

A thin film preparation method of the present invention is the method wherein a film supporting hyaluronic acid or a derivative thereof on a base film is formed and a carrier was further laminated thereon.

In the above-described thin film preparation method, it is preferable that the thickness of the film supporting hyaluronic acid or a derivative thereof on the base film is 10 to 500 nm.

In the above-described thin film preparation method, it is preferable that the carrier is a water-soluble polymer film or a cloth.

In the above-described thin film preparation method, it is preferable that a base film is initially prepared from a base film material, hyaluronic acid or a derivative thereof is supported on the base film by using a solution containing hyaluronic acid or a derivative thereof, and then a water-soluble film is prepared by adding dropwise a water-soluble polymer solution.

In the above-described thin film preparation method, it is preferable that a base film is prepared from a base film material, a film of hyaluronic acid or a derivative thereof is supported on the base film, a water-soluble polymer film is laminated on the obtained film, the water-soluble polymer film is removed by dissolution, and the thin film is transferred to a mesh or a non-woven fabric.

### EFFECT OF THE INVENTION

The thin film of the present invention is a laminate of (a) a base film supporting hyaluronic acid or a derivative thereof and (b) a carrier, wherein the thickness of film (a) is 10 to 500 nm; the invention can provide a thin film excellent in manageability, moisture-retaining effect, and unevenness correction effect. When the thin film of the present invention is used as a thin film cosmetic, fine wrinkles and lines can be hidden by smoothing the skin surface.

### BRIEF DESCRIPTION OF THE DRAWINGS

Fig. 1 shows the photos of hydrophilization of the thin film cosmetic of the present invention.
Fig. 2 shows the moisture content (rate of increase from the initial value) on the skin where the HA-supporting base film of the present invention was applied after the application of a moisturizer.
Fig. 3 shows the unevenness improvement effect of the HA-supporting base film (Preparation Example 1).
Fig. 4 shows the hiding effect of fine wrinkles of the HA-supporting base film (Preparation Example 1).
Fig. 5 shows the moisture content for the AHA-supporting base films with various film thicknesses.
Fig. 6 shows the moisture content of the stratum corneum of the HA-supporting base film (Preparation Example 2).
Fig. 7 shows a synergistic effect of the application of glycerin and the application of the HA-supporting base film (Preparation Example 2) on the skin.

### BEST MODE FOR CARRYING OUT THE INVENTION

The thin film of the present invention is a laminate of (a) a base film supporting hyaluronic acid or a derivative thereof and (b) a carrier, wherein the thickness of film (a) is 10 to 500 nm.

In the following, each component is described in detail.

### ((a) Base film supporting hyaluronic acid or a derivative thereof)

Hyaluronic acid is a kind of glycosaminoglycan discovered as the component of the vitreous body of the eye, and it is a linear macromolecular polysaccharide made of alternately connected D-N-acetylglucosamine and D-glucuronic acid. Hyaluronic acid has a property to form a gel with a large amount of water. Hyaluronic acid can be obtained by the conventional means such as extraction from hen's combs and fermentation method.

As hyaluronic acid, hyaluronate can also be used. Examples of hyaluronates include sodium hyaluronate and potassium hyaluronate.

Hyaluronic acid is commercially available as a cosmetic raw material, and the material normally used as the cosmetic raw material can be arbitrarily used in the present invention. From the standpoint of manageability, the material with the viscosity-average molecular weight of about 500K to 2M is preferable.

As a commercial sodium hyaluronate, Biohyalo-12 with the viscosity-average molecular weight of 1200K (manufactured by Shiseido Co., Ltd.) can be listed.

As a hyaluronic acid derivative, acetylated hyaluronic acid can be listed. In the present invention, the material commercially available as the cosmetic raw material can be arbitrarily used. From the standpoint of the easiness in handling during production, acetylated hyaluronic acid with the viscosity-average molecular weight of about 10K to 150K is preferable.

As a commercial acetylated hyaluronic acid, acetylated hyaluronic acid with the viscosity-average molecular weight of 100K (manufactured by Shiseido Co., Ltd.) can be listed.

In the present invention, it is preferable to use acetylated hyaluronic acid as the base film-supported hyaluronic acid or a derivative thereof. A thin film better in the moisture-retaining effect can be obtained by acetylated hyaluronic acid supported on the base film.

The base film material used in the present invention is not limited in particular; however, it is preferable to use one or more kinds of materials selected from the group consisting of polylactic acid, polyglycolic acid, polycaprolactone, and copolymers thereof.

Polylactic acid is called " biodegradable resin" , and it is known to degrade to carbon dioxide and water in compost in one week. Thus, it is getting attention because of its environment-friendliness. In addition, polylactic acid is useful because the mass production is possible from plant-derived raw materials such as corn and sweet potato and the cost is low.

It is necessary to prepare the (a) base film supporting hyaluronic acid or a derivative thereof (hereinafter referred to as "film (a)"), in the present invention, with a thickness of 10 to 500 nm. The thickness of film (a) is preferably 30 to 200 nm and especially preferably 100 to 180 nm.

If the thickness of film (a) is too thick, an uncomfortable feeling is caused at an application location and the hiding effect of fine wrinkles tends to be poor.

If the thickness of film (a) is too thin, the moisture-retaining effect and manageability tend to be poor.

The preparation method of film (a) is not limited in particular so far as the thickness of the present invention can be achieved. However, it is preferable to prepare by a spin-coating method, a (micro)gravure method, or a spray-coating method. In the case of spin coating, any spin coater can be used; however, it is preferable to carry out preparation at the rotation speed of 2000 to 5000 rpm.

The preparation of film (a) can be carried out by any optional method. For example, the method wherein a solution containing hyaluronic acid or a derivative thereof is dropwise added and dried after the preparation of the base film by a spin-coating method, a (micro)gravure method, a spray-coating method, etc.; the method wherein a dispersion liquid is dropwise added on the substrate that is used in a spin-coating method, a (micro)gravure method, a spray-coating method, etc. after obtaining the dispersion liquid in which hyaluronic acid or a derivative thereof is dispersed in the base film material; etc. can be listed.

The thin film of the present invention may be colored, to make it identifiable, with colorants, pigments, etc. that are normally used for cosmetics.

### ((b) Carrier)

The thin film of the present invention is obtained by further laminating the (b) carrier on the above-described (a) base film supporting hyaluronic acid or a derivative thereof (polylactic acid etc.). If only the (a) base film supporting hyaluronic acid or a derivative thereof is used, it is too thin, the removal after preparation is difficult, and the manageability is poor. However, a thin film excellent in manageability can be obtained by laminating the (b) carrier.

As (b) carrier, a water-soluble polymer film or a cloth is preferably used.

The thickness of the carrier is not limited in particular, but it is preferable to be 1 to 500 µm.

The water-soluble polymer film is not limited in particular, but it is preferable to use one or more polymer(s) selected from the group consisting of polyvinyl alcohol or a derivative thereof, a polyether or a derivative thereof, polysaccharides, a polymer electrolyte or a salt thereof, and hyaluronic acid or a derivative thereof.

Polyvinyl alcohol is obtained by the hydrolysis of polyvinyl acetate, and it is a linear polymer of vinyl alcohol. The molecular weight of polyvinyl alcohol is not limited in particular, but it is preferably 2,000 to 5,000.

In order to make the handling easy, PET (polyethylene terephthalate) resin etc. may be laminated as the (b) carrier in addition to the water-soluble polymer film when sodium hyaluronate is supported.

The cloth is not limited in particular, but it is preferable to use a mesh or a non-woven fabric.

The mesh in the present application means a sheet of network resin, and the examples of materials include PET resin, polyester resin, nylon, etc. From the standpoint of removability, it is preferable to use PET resin.

Handling can be improved by using a cloth as the (b) carrier.

The thin film of the present invention is preferably used as a thin film cosmetic. It is preferable to use the thin film cosmetic by pasting on the skin.

As the method in using the thin film cosmetic wherein a water-soluble polymer film is used as the (b) carrier, it is preferable to remove the water-soluble polymer film by dissolution immediately before application, apply the obtained base film on the skin, so that the face of hyaluronic acid or a derivative thereof directly contacts the skin, and retain it for a long time. The water-soluble polymer film can be dissolved, for example, by immersing in water.

In the present invention, a long time means 5 hours or longer.

By dissolving the water-soluble polymer film, only the very thin base film supporting hyaluronic acid or a derivative thereof can be applied on the skin.

As water-soluble polymer, hyaluronic acid or a derivative thereof can be used. For example, hyaluronic acid or a derivative thereof is formed into a thin film with the thickness of several micrometers to tens of micrometers and part of it is dissolved. Thus, it can be applied on the skin by transferring (scooping up) on a mesh or a cloth.

As the method in using a thin film cosmetic wherein a cloth is used as the (b) carrier, it is preferable to moisten the skin, apply the film (a) side of the thin film cosmetic on the skin, remove only the cloth, apply the obtained film (a) on the skin, and retain it for a long time.

The thin film cosmetic of the present invention does not generate an uncomfortable feeling after application; therefore, it can be used as a thin film cosmetic for daytime use rather than as the conventional pack cosmetic used on the bare skin for a short time in the evening or before going to bed. One can go out by wearing any optional cosmetic after application of the thin film cosmetic on the area of concern such as a dry area.

When used as the thin film cosmetic for daytime use, the thin film cosmetic can be peeled off with any makeup remover or face cleansing cosmetic at the same time when normal cosmetics are removed.

The shape of thin film cosmetic is not limited in particular so far as it is a shape that can easily be applied on the skin of the face, skin of the cheek, skin near the eyes, etc. However, it is preferable to use a round shape, oval shape, magatama shape, etc.

The area of thin film cosmetic is preferably 10 mm² to 100 cm² and more preferably 4 cm² to 25 cm².

It is also preferable to use the thin film of the present invention as general goods for makeup or beauty supplies.

### EXAMPLES

Initially, the preparation method of the thin film of the present invention will be explained. As the preparation method of thin film, the below-described Preparation Examples 1 to 3 can be listed. In all the evaluation test examples, hyaluronic acid or a derivative thereof on the (a) base film supporting hyaluronic acid or a derivative thereof was applied on the skin so that it contacts the skin.

### Preparation Example 1) Hyaluronic acid-supporting thin film (spin-coating method)

All operations were carried out by setting up a spin coater (Opticoat MS-A 150, manufactured by MIKASA Co., Ltd.) inside a clean room (class: 10,000).

Silicon substrate (manufactured by KST World Corp.) was cut into 4 cm × 4 cm, immersed in SPM (H₂SO₄/H₂O₂ = 2.3:1 (v/v)) at 120 °C for 10 minutes, and then washed with ion-exchanged water (specific resistance: 18 MΩcm). This substrate was placed on a spin coater, and the spin coating (4000 rpm, 20 seconds) was carried out by adding dropwise 500 µL of dichloromethane solution of polylactic-L-acid (hereinafter referred to as PLA) (Mw: 100,000, manufactured by Polysciences Inc., 10 mg/mL) to obtain a base film.

Subsequently, 5 mg/mL, 10 mg/mL, or 20 mg/mL sodium hyaluronate (Biohyalo 12 (manufactured by Shiseido Co., Ltd.), hereinafter referred to as HA) was prepared with 50% and 70% ethanol aqueous solutions. About 1.5 mL of this was dropwise added on the base film and dried (80 °C, 30 minutes), the substrate surface was washed with ion-exchanged water (room temperature, 1 minute), and the surface was dried with nitrogen gas; thus (a) a HA-supporting base film was obtained.

The thickness of each (a) HA-supporting base film, obtained as described above, was measured with an atomic force microscope (manufactured by Keyence Corporation); it was 70 nm.

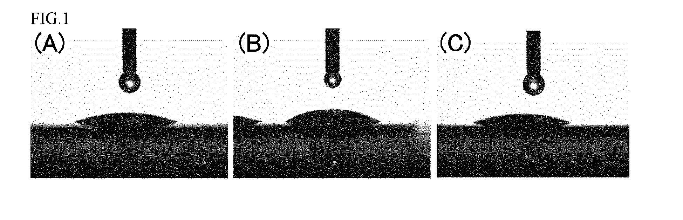

In addition, the contact angles of the obtained HA-supporting base films were measured; the contact angle of the HA-supporting base film prepared from 10 mg/mL HA in 50% ethanol aqueous solution was 25±8° (Fig. 1(A)), that of the HA-supporting base film prepared from 20 mg/mL HA in 50% ethanol aqueous solution was 25±2° (Fig. 1(B)), that of the HA-supporting base film prepared from 0.5 mg/mL HA in 70% ethanol aqueous solution was 20±1° (Fig. 1(C)), and that of the HA-supporting base film prepared from 10 mg/mL HA in 70% ethanol aqueous solution was 22±4°. The contact angle of the base film itself is 72±1°; therefore, it was confirmed that hydrophilization was achieved by the coating of HA.

In addition, 0.5 mL of polyvinyl alcohol aqueous solution (hereinafter PVA, Mw: 22,000, manufactured by Kanto Chemical Co., Inc., 100 mg/mL) was dropwise added on the surface of the prepared HA-supporting base film and dried; thus a PVA film was formed on the HA-supporting base film (80 °C, 30 minutes). A thin film was obtained by peeling off, from the silicon substrate, the HA-supporting base film together with the PVA film.

The below-described effects were evaluated by applying, on the skin, the HA-supporting base film, which was obtained by dissolving the PVA film by immersing the thin film in water.

Preparation Example 2) Hyaluronic acid-supporting thin film (microgravure method)

A PVA aqueous solution was spread on an olefin/PET film, poly-DL-lactic acid (hereinafter referred to as PDLLA) (Mw: 450,000, manufactured by Polysciences Inc.) in ethyl acetate solution was deposited thereon, and sodium hyaluronate was lastly spread to obtain a thin film.

The thickness of each (a) HA-supporting base film, obtained as described above, was measured with an atomic force microscope (manufactured by Keyence Corporation); it was 120 nm.

The below-described effects were evaluated by applying, on the skin, the HA-supporting base film, which was obtained by dissolving the PVA film by immersing in water after the olefin/PET film was peeled from the thin film.

### Preparation Example 3) Acetylated hyaluronic acid-supporting thin film (spin-coating method)

On a silicon wafer, 1 wt % acetylated hyaluronic acid (acetylated hyaluronic acid with a viscosity-average molecular weight of 100K: hereinafter referred to as AHA) in 70% ethanol solution was cast and dried at 60 °C for 15 minutes. On that, 1 wt % PDLLA ethyl acetate solution was spin-coated at 4000 rpm for 20 seconds and dried at 60 °C for 15 minutes.

Subsequently, the AHA-supporting base film was peeled off from the silicon wafer in water. A mesh was floated in water, the mesh was positioned to the side of the base film (PDLLA), and the AHA-supporting base film was scooped up.

The effects described below were evaluated by moistening the skin, applying the AHA side on the skin, and peeling off only the mesh; thus by the application of the HA-supporting base film on the skin.

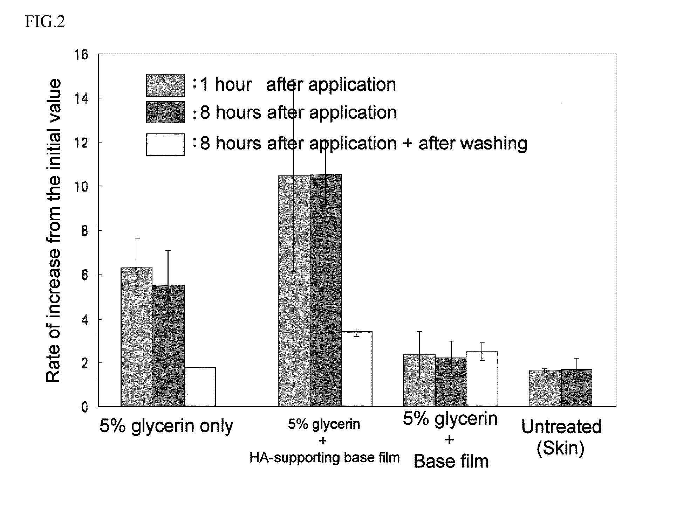

The present inventors investigated the moisture-retaining effect of the thin film obtained in the above-described Preparation Example 1 (the thickness of film (a): 70 nm). As the test method, the below-described Method 1 was used. The results are shown in Figure 2.

### •Test Method 1 of the moisturizing effect

The thin film obtained in Preparation Example 1 was immersed in water, the PVA film was dissolved, and the HA-supporting base film was obtained. The thin film without HA was immersed water, the PVA film was dissolved, and the base film (base film without HA) was obtained. Subsequently, various base films were applied on the forearm region, and the time-dependent moisture-retaining effect was measured.

The moisture content was measured with a SKICON-200 (manufactured by I.B.S. Co., Ltd.) when only 5% glycerin aqueous solution was applied (Test Example 1), when the HA-supporting base film (Preparation Example 1) was applied on the skin after the application of 5% glycerin aqueous solution (Test Example 2), when, the base film (base film without HA) was applied on the skin after the application of 5% glycerin aqueous solution (Test Example 3), and when nothing was applied (Test Example 4).

The measurement was carried out at application, at 1 hour after application, at 8 hours after application, and at 8 hours after application but after washing. The measured values were calculated as the rate of increase from the initial value (at application).

According to Fig. 2, the moisture content increased dramatically by using 5% glycerin and the HA-supporting base film of the present invention in combination; thus it was clarified that it has a time-dependent drying suppression effect.

When 5% glycerin and the base film (base film without HA) were used in combination, an increase in the moisture content was hardly observed compared with the case of the HA-supporting base film of the present invention.

Thus, the HA-supporting base film of the present invention has a prominent moisture-retaining effect.

Subsequently, the present inventors investigated the unevenness correction effect of the thin film (the thickness of film (a): 120 nm) obtained in the above-described Preparation Example 2.

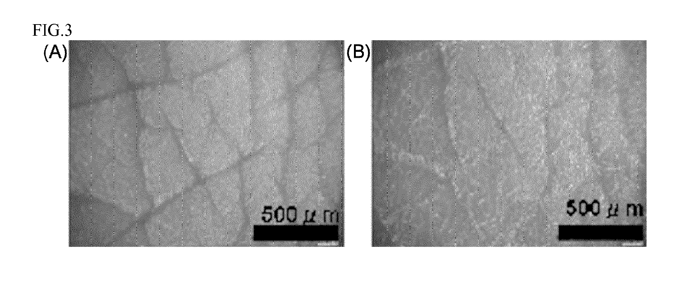

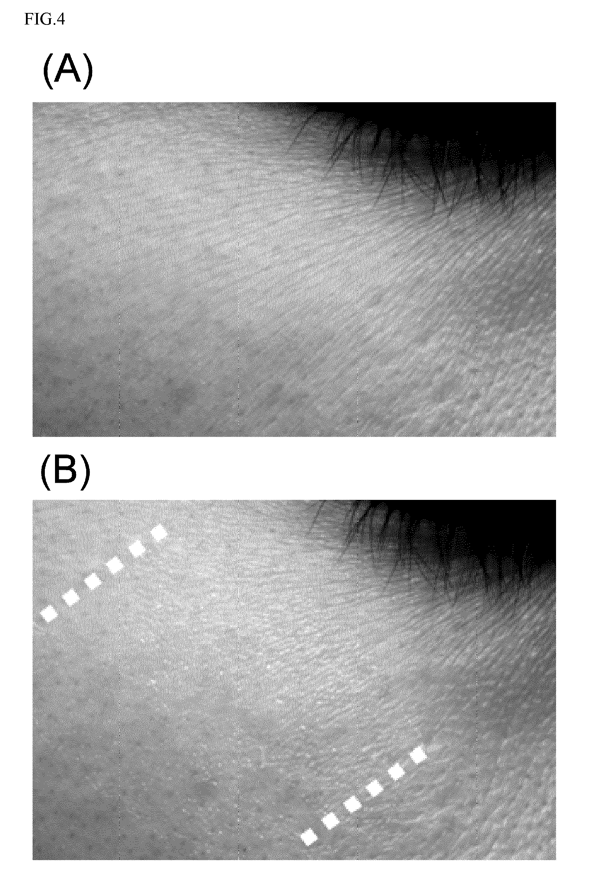

The HA-supporting base film of Preparation Example 2 was applied around the eye of a subject. The results observed with a digital microscope are shown in Fig. 3. In addition, the photographic results are shown in Fig. 4. The photo (A) is for the bare skin, and the photo (B) is after applying the HA-supporting base film on the bare skin. Dotted lines in Fig. 4 indicate both ends of the HA-supporting base film.

From Fig. 3, it was found that the skin unevenness became less prominent by the application of the HA-supporting base film.

It was also found, from Fig. 4, that the fine wrinkles at the section where the HA-supporting base film was applied became less prominent. This hiding effect of fine wrinkles was maintained even 4 hours after application.

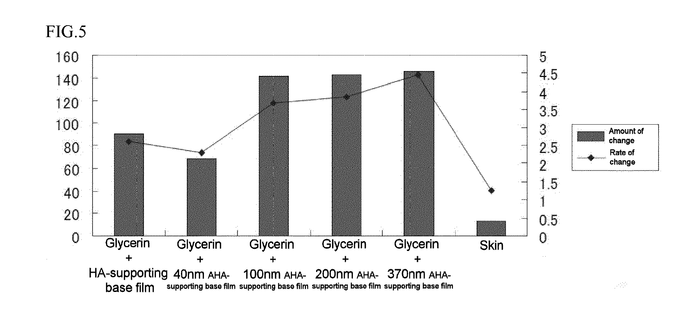

Subsequently, the present inventors carried out the moisture-retaining effect test for the thin film prepared in Preparation Example 1 (the thickness of film (a): 70 nm) or the thin film prepared in Preparation Example 3 (the thickness of film (a): 40 to 370 nm). As the test method, the below-described Method 2 was used. The results are shown in Figure 5.

### • Test Method 2 of the moisturizing effect

After the application of the defined amount of 5% glycerin (3.75 µl/cm²) on the skin, the HA-supporting base film (Preparation Example 1) or the AHA-supporting base film (Preparation Example 3) was applied. At 6 hours after application but after the film removal by tape stripping, the moisture content of the stratum corneum was measured with a SKICON-200 (manufactured by I.B.S. Co., Ltd.). The measurement was carried out at five locations on the inner side of the forearm of three panelists.

From Fig. 5, it was clarified that the AHA-supporting base film has a higher moisture-retaining effect than the HA-supporting base film.

Accordingly, it is preferable to use the base film supporting acetylated hyaluronic acid as film (a) in the thin film of the present invention.

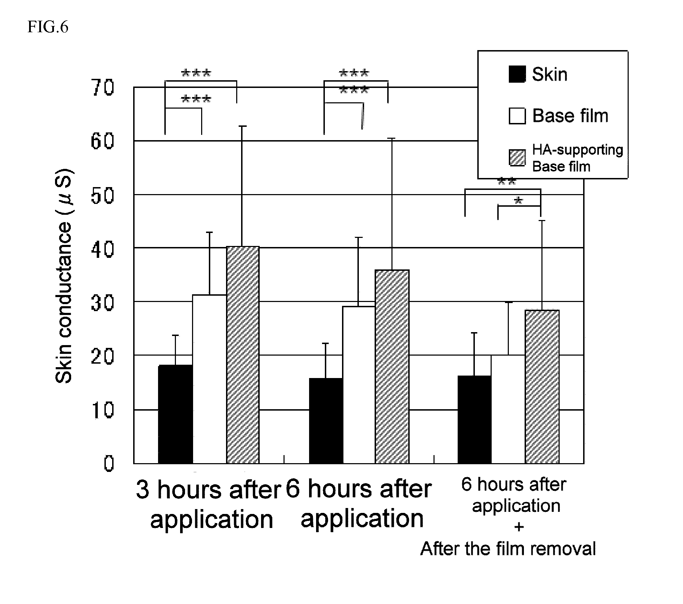

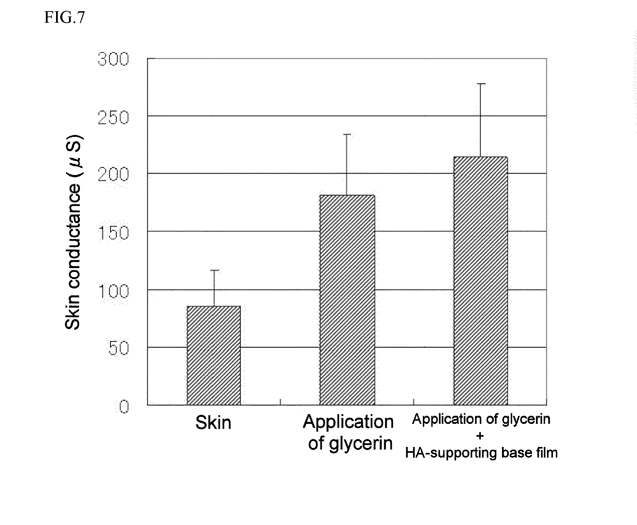

Subsequently, the present inventors carried out the moisture-retaining effect tests for the thin film prepared in Preparation Example 2. As the test method, the below-described Methods 3 and 4 were used. The results are shown in Figures 6 and 7, respectively.

### •Test Method 3 of the moisturizing effect

Water (3.75 mg/cm²) was applied on the skin in advance, and the HA-supporting base film (Preparation Example 2) or the base film (base film without HA) was applied on that. At 3 hours after application, at 6 hours after application, and at 6 hours after application but after the film removal by water washing, the moisture content of the stratum corneum was measured with a SKICON-200 (manufactured by I.B.S. Co., Ltd.). The measurement was carried out, seven times in total, at the inner side of the forearm of three panelists.

### • Test Method 4 of the moisturizing effect

5% Glycerin aqueous solution (3.75 mg/cm²) was applied on the skin in advance, and the HA-supporting base film (Preparation Example 2) was applied on that. After 6 hours, the HA-supporting base film was removed by tape stripping.

The moisture content of the stratum corneum was measured with a SKICON-200 (manufactured by I.B.S. Co., Ltd.) for the bare skin, for the skin on which 5% glycerin aqueous solution was applied, and for the skin on which 5% glycerin aqueous solution and the HA-supporting base film were applied. The measurement was carried out, five times in total, at the inner side of the forearm of six panelists.

According to Fig. 6, the moisture content of the skin increased by the application of the base film or the HA-supporting base film compared with the bare skin. Compared with the base film, the average moisture content was higher for the HA-supporting base film, and the moisture content of the skin where the HA-supporting base film was removed by washing also had a trend to increase.

Accordingly, it was found that the HA-supporting base film obtained from the thin film of the present invention achieves a skin care effect by moisture retention, when applied on the skin, and the skin condition can be improved.

According to Fig. 7, the moisture content increased by using a glycerin aqueous solution and the HA-supporting base film in combination; thus an excellent moisture-retaining effect could be achieved.

Accordingly, in order to achieve an additional moisture-retaining effect when the thin film of the present invention is used as a thin film cosmetic, it is preferable to apply the thin film cosmetic after the application of any optional cosmetic wherein a moisturizer is blended (for example, lotion etc.).

## Claims

1. A thin film wherein (a) a base film supporting hyaluronic acid or a derivative thereof and (b) a carrier are laminated and the thickness of film (a) is 10 to 500 nm.

2. The thin film according to claim 1, wherein the (b) carrier is a water-soluble polymer film or a cloth.

3. The thin film according to claim 1 or 2, wherein the thickness of film (a) is 30 to 200 nm.

4. The thin film according to any one of claims 1 to 3, wherein the base film comprises polylactic acid, polyglycolic acid, polycaprolactone, or a copolymer thereof.

5. The thin film according to any one of claims 1 to 4, wherein the thin film is formed by the method selected from the group consisting of a spin-coating method, a gravure method, a microgravure method, and a spray-coating method.

6. The thin film according to any one of claims 2 to 5, wherein the water-soluble polymer film is selected from the group consisting of polyvinyl alcohol or a derivative thereof, a polyether or a derivative thereof, polysaccharides, and a polymer electrolyte or a salt thereof.

7. The thin film according to any one of claims 2 to 5, wherein the cloth is a non-woven fabric or a mesh.

8. A thin film cosmetic comprising the thin film according to any one of claims 1 to 7.

9. An unevenness-improving thin film cosmetic comprising the thin film cosmetic according to the claim 8.

10. A thin film cosmetic for use near the eye and near the mouth comprising the thin film cosmetic according to claim 8.

11. A method in using the thin film cosmetic according to claim 8, wherein the water-soluble polymer film is removed by dissolution immediately before application, the obtained base film supporting hyaluronic acid or a derivative thereof is applied on the skin, so that hyaluronic acid or a derivative thereof contacts the skin, and retained for a long time.

12. A method in using the thin film cosmetic according to claim 8, wherein the skin is moistened, the thin film cosmetic is applied, only the cloth is peeled off, the obtained base film supporting hyaluronic acid or a derivative thereof is applied on the skin, so that hyaluronic acid or a derivative thereof contacts the skin, and is retained for a long time.

13. A beauty treatment method wherein the thin film cosmetic according to claim 8 is applied on the skin.

14. A beauty treatment method comprising a step of applying one or more kinds of cosmetics selected from the group consisting of lotion, milky lotion, beauty essence, and cream and a step of applying the thin film cosmetic according to claim 8.

15. A cosmetic kit comprising the thin film cosmetic according to claim 8 and one or more kinds of cosmetics selected from the group consisting of lotion, milky lotion, beauty essence, and cream.

16. A thin film preparation method wherein a film supporting hyaluronic acid or a derivative thereof on a base film is formed and a carrier was further laminated thereon.

17. The thin film preparation method according to claim 16, wherein the thickness of the film supporting hyaluronic acid or a derivative thereof on the base film is 10 to 500 nm.

18. The thin film preparation method according to claim 16 or 17, wherein the carrier is a water-soluble polymer film or a cloth.

19. The thin film preparation method according to claim 18, wherein a base film is initially prepared from a base film material, hyaluronic acid or a derivative thereof is supported on the base film by using a solution containing hyaluronic acid or a derivative thereof, and then a water-soluble film is prepared by adding dropwise a water-soluble polymer solution.

20. The thin film preparation method according to claim 18, wherein a base film is prepared from a base film material, a film of hyaluronic acid or a derivative thereof is supported on the base film, a water-soluble polymer film is laminated on the obtained film, the water-soluble polymer film is removed by dissolution, and the thin film is transferred to a mesh or a non-woven fabric.
